# DEMANDE DE BREVET EUROPEEN

(11) **EP 4 843 600 A1**
(43) Date de publication de la demande: **25.11.2026**
(21) Numéro de dépôt: 26156135.1
(22) Date de dépôt: 03.02.2026
(51) Int. Cl.: A61M 16/20, A61M 16/00

(54) **APPAREIL D ASSISTANCE À LA TOUX POUR OPÉRER DES INSUFFLATIONS ET EXSUFFLATIONS DE GAZ**

(30) Priorité: 21.05.2025 FR 2505438
(71) Demandeur: EOVE, 64000 Pau (FR); Air Liquide Medical Systems, 92182 Antony Cedex (FR)
(72) Inventeur: LEBRET, Marius, 92182 Antony Cedex (FR); GALBRUN, Marie-Amedee, 64000 Pau (FR); JOURDAIN, Cedric, 64000 Pau (FR); VENKATAPEN, Delphine, 92182 Antony Cedex (FR)
(74) Mandataire: Air Liquide

(57) **Abrégé**

L'invention concerne un appareil d'insufflation et d'exsufflation de gaz (1) comprenant un générateur de flux (2) ; un circuit de gaz (3, 4, 8) avec lignes d'insufflation (3) et d'exsufflation (4), raccordées à un tronçon de jonction (8) commun alimentant une interface respiratoire (10) ; des moyens de contrôle de flux (5) ; des moyens de pilotage (6) ; et des moyens de mesure de pression (7). Les moyens de pilotage (6) sont configurés pour déclencher une phase de pause respiratoire à la fin d'une phase d'insufflation ; et détecter l'existence d'un phénomène indésirable en comparant la pression de pause à une pression de référence.

## Description

L'invention concerne un appareil d'insufflation et d'exsufflation de gaz, aussi appelé « appareil d'assistance à la toux » ou plus simplement « appareil à toux », permettant de détecter des phénomènes indésirables susceptibles de nuire à l'efficacité d'une thérapie par insufflation-exsufflation de gaz afin de rendre la délivrance du traitement au patient plus efficace.

La thérapie par insufflation-exsufflation (appelée « INEX »), mise en œuvre par d'un in-exsuflateur mécanique, c'est-à-dire un appareil d'insufflation et d'exsufflation de gaz, repose sur une délivrance cyclique (i.e. cycles « INEX ») sur une durée brève, typiquement de moins de 15 secondes pour un cycle complet, chaque cycle comprenant au moins : une phase d'insufflation, d'une pression inspiratoire positive (i.e. gaz sous pression) dans les voies aériennes d'une personne à traiter, c'est-à-dire un patient, suivi d'une phase d'exsufflation à pression négative, c'est-à-dire avec aspiration du gaz hors des voies aériennes du patient. Une phase de pause optionnelle peut s'intercaler entre les phases d'insufflation et d'exsufflation.

En répétant ces phases sur plusieurs cycles d'affilée, on peut mimer le mécanisme de la toux.

L'objectif principal de ce traitement est de palier des efforts de toux inefficaces chez des patients présentant un encombrement et une faiblesse musculaire. Ainsi, cette thérapie est communément proposée aux patients ayant une maladie neuromusculaire lente à progressive et/ou aux patients paralytiques d'étiologie traumatique, et ce, chez des patients allant des petits enfants aux adultes.

L'in-exsuflateur mécanique, aussi appelé « appareil d'assistance à la toux » ou « appareil à toux », est raccordé au patient par l'intermédiaire d'un circuit de gaz, typiquement un ou plusieurs conduits, passages, tuyaux ou analogue, à une interface respiratoire oro-nasale, typiquement un masque facial, recouvrant la bouche et le nez du patient à traiter. Des exemples de tels appareils à toux sont donnés par EP3622991 et EP3622992.

Utiliser un appareil à toux pour traiter de nombreux patients peut s'avérer crucial car il réduit le nombre d'infection pulmonaires pouvant survenir chez ces patients, améliore leur qualité de vie et/ou peut réduire leur taux ou risque d'hospitalisation. Toutefois, l'existence de phénomènes indésirables est un inconvénient majeur à l'efficacité du traitement d'insufflation-exsufflation (INEX) pour l'aide à la toux.

Ainsi, les fuites gazeuses au niveau de l'interface respiratoire posent problème. En effet, lorsque l'interface respiratoire, i.e. masque, n'est pas étanche, une partie du volume insufflé s'échappe (i.e., fuites) à l'atmosphère ambiante et les voies aériennes, i.e. supérieures et pulmonaires, du patient sont susceptibles de ne pas atteindre la pression souhaitée en fin de phase d'insufflation, c'est-à-dire la pression fixée par le personnel soignant et jugée efficace pour le traitement du patient considéré. Il s'ensuit alors une réduction du pic de débit expiratoire du patient et donc une réduction de l'efficacité globale du traitement.

Un autre phénomène indésirable susceptible de se produire est la fermeture de la glotte du patient pendant l'insufflation de l'air en cours de traitement. En effet, l'efficacité du traitement est dépendant de la bonne ouverture des voies aériennes du patient pendant l'insufflation de l'air. Si la glotte du patient se ferme au cours de l'insufflation, le poumon est insuffisamment rempli et n'atteint pas, là encore, la pression d'insufflation souhaitée qui est réglée sur le dispositif d'aide à la toux, i.e. l'appareil à toux. L'efficacité du traitement s'en trouve alors réduite, voire annulée.

Il en va de même si le temps inspiratoire réglé sur l'appareil à toux, i.e. durée d'insufflation, est trop court par rapport au temps nécessaire pour que la pression des voies aériennes du patient atteigne celle réglée.

Certains dispositifs d'aide à la toux affichent les courbes de débit inspiratoire pour s'assurer qu'il n'y a pas de fuites, de fermeture glottique ou autre phénomène indésirable. Mais cette solution n'est pas idéale car l'utilisateur doit pouvoir lire les courbes sur l'écran où elles s'affichent (i.e. appareil à toux, un ordinateur...) et surtout être en mesure de les interpréter correctement, c'est-à-dire sans commettre d'erreur.

Or, il peut être difficile de faire la différence entre des artéfacts liés aux différents aspects de la physiologie ou du comportement du patient (e.g. mouvements, toux...) qui influent sur la forme du débit insufflé et des évènements indésirables, tels que des fuites, une fermeture glottique ou autre.

De plus, cette analyse de courbes prend du temps, complexifie la prise en charge du patient et ne peut être réalisée que par un professionnel de santé formé et expérimenté dans ce domaine.

D'une façon générale, étant donné que tous ces phénomènes indésirables (i.e. fuites, fermeture glottique...) peuvent entrainer une réduction significative de l'efficacité du traitement du patient, un problème est de pouvoir les détecter de manière efficace et fiable, afin de rendre la délivrance du traitement par insufflation-exsufflation (INEX) la plus efficace possible, même lorsque le patient est soigné à son domicile, sans présence à ses côtés d'un professionnel de santé formé et expérimenté en analyse de courbes.

En d'autres termes, un but de l'invention est de proposer un appareil d'insufflation et d'exsufflation de gaz amélioré permettant de détecter un ou des phénomènes indésirables de type fuites au masque, fermeture de glotte ou autres, susceptibles de nuire à l'efficacité d'une thérapie par insufflation-exsufflation de manière à rendre la délivrance du traitement au patient plus efficace.

Une solution de l'invention concerne alors un appareil d'insufflation et d'exsufflation de gaz, aussi appelé in-exsuflateur, appareil d'assistance à la toux ou appareil à toux, comprenant :
- un générateur de flux configuré pour insuffler et/ou exsuffler du gaz,
- un circuit de gaz comprenant une ligne d'insufflation pour acheminer du gaz délivré par le générateur de flux, pendant au moins une phase d'insufflation de gaz, et une ligne d'exsufflation pour acheminer du gaz jusqu'au générateur de flux, pendant au moins une phase d'exsufflation de gaz, lesdites lignes d'insufflation et d'exsufflation étant raccordées fluidiquement à un tronçon de jonction commun configuré pour être raccordé fluidiquement (i.e. en un site de raccordement) à un conduit (i.e. tuyau) flexible alimentant une interface respiratoire,
- des moyens de contrôle de flux agencés sur ledit circuit de gaz, configurés pour contrôler la circulation du gaz dans au moins lesdites lignes d'insufflation et d'exsufflation,
- des moyens de pilotage comprenant une électronique de commande configurés pour contrôler le générateur de flux ; et les moyens de contrôle de flux, et
- des moyens de mesure de pression configurés pour mesurer la pression du gaz dans le circuit de gaz entre les moyens de contrôle de flux et le site de raccordement fluidique au conduit flexible, et fournir ladite au moins une mesure de pression aux moyens de pilotage.

Selon l'invention, les moyens de pilotage de l'appareil d'insufflation et d'exsufflation de gaz sont en outre configurés pour :
a) déclencher une phase de pause respiratoire à la fin d'au moins une phase d'insufflation en contrôlant (i.e. commandant ou pilotant) les moyens de contrôle de flux pour stopper ou empêcher (i.e. interdire, interrompre etc....) toute circulation de gaz au sein desdites lignes d'insufflation et d'exsufflation,
b) comparer au moins une pression de pause (Pp) mesurée par les moyens de mesure de pression pendant ladite pause respiratoire, à une pression de référence (Pr) fixée par l'utilisateur, et
c) détecter l'existence d'un phénomène indésirable, lorsque ladite au moins une pression de pause (Pp) mesurée est inférieure à la pression de référence (Pr) ou à une proportion de ladite pression de référence (Pr) fixée par l'utilisateur.

Autrement dit, grâce à l'appareil d'insufflation et d'exsufflation de gaz de l'invention, on peut mesurer, pendant une phase de pause inspiratoire, la pression réelle (Pp) des voies aériennes du patient, notamment celles de ses poumons, et la comparer à la pression réglée (Pr), détecter l'existence d'un phénomène indésirable susceptible d'engendrer une perte d'efficacité du traitement INEX.

Ayant connaissance du phénomène indésirable détecté, l'utilisateur, c'est-à-dire le personnel soignant, peut intervenir pour corriger le problème engendrant le phénomène indésirable, par exemple en ajustant ou appuyant sur le masque pour améliorer son étanchéité, augmenter le temps insufflatoire et/ou réduire la pente inspiratoire ou la pression inspiratoire de manière à éviter ou minimiser la fermeture glottique.

Selon le mode de réalisation considéré, l'appareil d'insufflation et d'exsufflation de gaz de l'invention peut comprendre l'une ou plusieurs des caractéristiques suivantes :
- les moyens de pilotage sont en outre configurés pour avertir l'utilisateur de la détection d'un phénomène indésirable, c'est-à-dire en réponse à la détection d'un tel phénomène indésirable.
- les moyens de pilotage comprennent une électronique de commande comprenant un ou des microprocesseur(s) ou microcontrôleur(s).
- les moyens de pilotage sont configurés pour commander, contrôler ou piloter le générateur de flux, pendant un ou des cycles successifs d'insufflation et d'exsufflation de gaz, chaque cycle comprenant une phase d'insufflation de gaz et une phase d'exsufflation de gaz, et éventuellement phase de pause respiratoire entre au moins une phase d'insufflation de gaz et une phase d'exsufflation de gaz.
- les moyens de pilotage sont configurés pour contrôler les moyens de contrôle de flux pour autoriser ou interdire une circulation de gaz dans lesdites lignes d'insufflation et d'exsufflation.
- les moyens de pilotage sont configurés pour commander, contrôler ou piloter les moyens de contrôle de flux pour autoriser ou interdire une circulation de gaz dans lesdites lignes d'insufflation et d'exsufflation en fonction des phases d'insufflation de gaz, d'exsufflation de gaz et éventuellement de pause respiratoire.
- les moyens de pilotage sont configurés pour commander, contrôler ou piloter les moyens de contrôle de flux pour isoler fluidiquement une partie aval du circuit de gaz, pendant la phase de pause.
- la partie aval du circuit de gaz comprend des portions des lignes d'insufflation et d'exsufflation et le tronçon de jonction commun.
- le tronçon de jonction commun est situé à une sortie du circuit de gaz, c'est-à-dire à son extrémité aval.
- le gaz insufflé et le gaz exsufflé transitent par le tronçon de jonction commun lors des phases d'insufflation et d'exsufflation.
- le générateur de flux comprend une turbine motorisée, de préférence la turbine motorisée délivre un flux d'air.
- la turbine motorisée comprend un moteur électrique.
- la turbine motorisée comprend une électronique de commande du moteur.
- la turbine motorisée peut être associée à une ou des vannes ou valves, par exemple des électrovannes ou des vannes pneumatiques.
- les moyens de pilotage sont configurés pour contrôler l'électronique de commande du moteur afin de moduler ou modifier la pression ou le débit de gaz, i.e. d'air, délivré par la turbine.
- il comprend des moyens de raccordement configurés pour permettre un raccordement fluidique d'un conduit ou tuyau flexible raccordé fluidiquement à une interface respiratoire
- le conduit ou tuyau flexible est en polymère.
- les moyens de raccordement sont agencés à la sortie du tronçon de jonction commun du circuit de gaz.
- les moyens de raccordement sont configurés pour permettre un raccordement par vissage, par emboitement ou autre du tuyau flexible.
- l'interface respiratoire est un masque respiratoire facial, une sonde de trachéostomie ou un embout buccal.
- un tuyau flexible est raccordé aux moyens de raccordement de l'appareil.
- une interface respiratoire est raccordée au tuyau flexible.
- la pause respiratoire a une durée de pause comprise entre 100 msec et 1 sec, de préférence moins de 800 ms, de préférence encore moins de 600 ms.
- la pause respiratoire a une durée de pause typiquement comprise entre 150 et 400 msec.
- les moyens de pilotage sont configurés pour détecter un phénomène indésirable lorsque ladite au moins une pression de pause (Pp) mesurée est inférieure ou égale à 95% de la pression de référence (Pr) fixée par l'utilisateur, typiquement entre 50 et 90% (i.e. valeur de seuil ; x%), par exemple une valeur de seuil (x%) de l'ordre de 80% de la pression de référence (Pr).
- la valeur de seuil (x%) peut être réglable ou réglée par l'utilisateur.
- il comprend un écran d'affichage.
- l'écran tactile, c'est-à-dire à dalle tactile.
- l'écran est à affichage en couleurs.
- les moyens de pilotage sont configurés pour contrôler ou opérer un affichage d'une information ou alerte de détection de phénomène indésirable sur ledit écran d'affichage.
- l'affichage de l'information ou de l'alerte de détection peut prendre différentes formes comme l'affichage d'un message d'avertissement alphanumérique ou autre, par exemple un code d'alerte, d'une animation vidéo, d'une icône ou fenêtre d'alerte dédiée ou autre.
- l'affichage de l'information ou de l'alerte de détection comprend un affichage d'un niveau de baisse de pression (i.e. un %) ou de toute autre valeur ou grandeur permettant d'exprimer la perte de performance.
- la ligne d'insufflation est raccordée fluidiquement à une sortie de gaz du générateur de flux, i.e. de la turbine.
- la ligne d'exsufflation raccordée fluidiquement à l'entrée de gaz du générateur de flux, i.e. de la turbine.
- les moyens de contrôle de flux comprennent une ou plusieurs vannes, typiquement une ou des électrovannes, de préférence associées à une ou des vannes pneumatiques.
- il comprend des moyens de réglage de pression configurés pour permettre à l'utilisateur de régler ou fixer ladite pression de référence (Pr).
- il comprend des moyens de réglage de valeur de seuil (x%).
- les moyens de réglage de pression ou de valeur de seuil (x%) comprennent une ou des touches tactiles affichées sur l'écran d'affichage.
- alternativement, les moyens de réglage de pression ou de valeur de seuil (x%) comprennent un ou des boutons, par exemple rotatifs, curseurs ou analogues.
- l'écran d'affichage fait partie d'une interface homme-machine (IHM) de l'appareil.
- il comprend des moyens de mémorisation.
- les moyens de mémorisation comprennent une mémoire flash, EEPROM ou analogue.
- les moyens de pilotage sont configurés pour mémoriser au sein des moyens de mémorisation, la valeur de pression réglée ou fixée et/ou la valeur de seuil (x%) réglée ou fixée.
- les moyens de pilotage sont configurés pour afficher de manière différée la ou les phénomènes indésirables ayant été détectés pendant le déroulé d'un traitement INEX d'un patient, par exemple lors d'un post-traitement après traitement INEX.
- le post-traitement après traitement INEX peut se faire à partir d'un enregistrement de la ou des courbes de pression. En combinant la (les) courbe de pression avec celle de débit, on peut affiner l'identification du phénomène (i.e. fuites, temps d'insufflation trop court, fermeture glottique).
- les phénomènes indésirables sont choisis parmi une fuite gazeuse (au masque), une fermeture de la glotte du patient (pendant l'insufflation) et un temps inspiratoire réglé trop court.
- la pression de référence (Pr) correspond à la valeur de pression réglée (et mémorisée), c'est-à-dire au réglage de la pression d'insufflation.
- la pression réglé (Pr) est typiquement comprise entre 5 et 70 cmH2O.
- la durée d'un cycle INEX est inférieure ou égale à 15 secondes, de préférence comprise entre 3 et 10 secondes, par exemple de l'ordre de 6 secondes.
- selon un mode de réalisation, le générateur de flux et les moyens de contrôle de flux forment ou font partie d'un système intégré.
- selon un autre mode de réalisation, le générateur de flux et les moyens de contrôle de flux sont indépendants.
- selon un mode de réalisation, les moyens de pilotage sont en outre configurés pour analyser la forme d'un signal de pression pendant la phase de pause inspiratoire et en déduire au moins une cause possible de phénomène indésirable.
- les moyens de pilotage sont en outre configurés pour commander un affichage sur les moyens d'affichage, de ladite cause possible de phénomène indésirable.
- la cause possible de phénomène indésirable est choisie parmi une fermeture glottique, un remplissage incomplet des voies aériennes du patient ou une (des) fuite(s), ou une autre cause possible.
- les moyens de pilotage sont configurés pour contrôler ou opérer un affichage d'une information ou alerte de cause possible de phénomène indésirable sur l'écran d'affichage.

D'une façon générale, dans le cadre de l'invention, les termes « moyens de » peuvent être remplacés ou substitués par les termes « dispositif de » qui sont considérés comme strictement équivalent, par exemple « moyens de mesure » équivalent à « dispositif mesure », etc...

L'invention va maintenant être mieux comprise grâce à la description détaillée suivante, faite à titre illustratif mais non limitatif, en référence aux figures annexées parmi lesquelles :
Fig. 1 schématise un mode de réalisation d'un appareil d'insufflation et d'exsufflation de gaz selon l'invention.
Fig. 2 schématise une première courbe de pression lors d'un cycle d'insufflation - exsufflation comprenant une phase de pause inspiratoire.
Fig. 3 schématise une seconde courbe de pression lors d'un cycle d'insufflation - exsufflation comprenant une phase de pause inspiratoire.

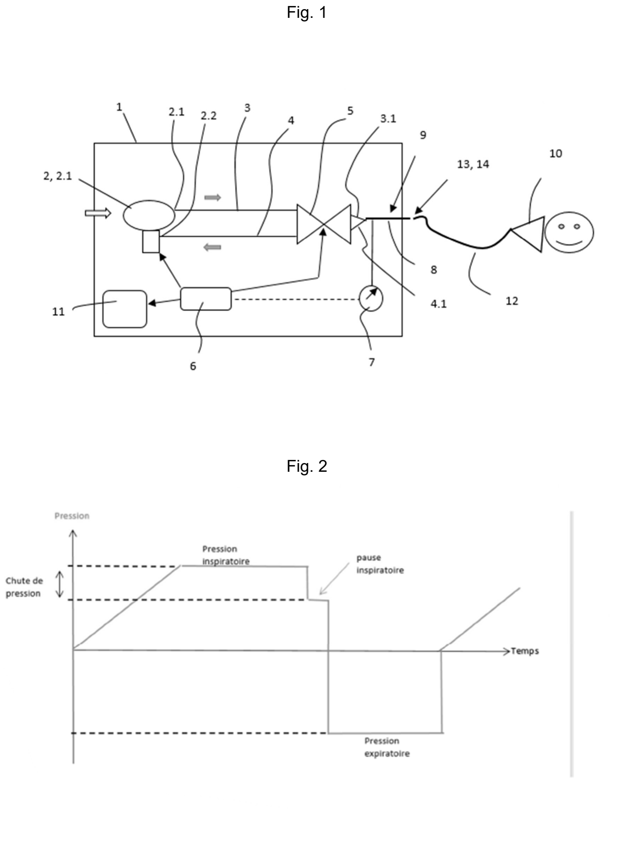

Fig. 1 schématise un mode de réalisation d'un appareil d'insufflation et d'exsufflation 1 de gaz selon l'invention, aussi appelé in-exsufflateur mécanique, appareil à toux ou analogue, fonctionnant selon des cycles d'insufflation-exsufflation de gaz ou cycles « INEX ». Chaque cycle INEX comprend une phase d'insufflation de gaz suivie d'une phase d'exsufflation de gaz, et éventuellement une phase de pause intercalée entre les phases d'insufflation et d'exsufflation de gaz.

Ainsi, Fig. 2 schématise une courbe de pression lors d'un cycle d'insufflation/exsufflation de gaz comprenant une phase de pause inspiratoire intercalée entre les phases d'insufflation et d'exsufflation de gaz.

En cas de phénomène indésirable, on constate une chute de pression en début de pause inspiratoire.

La durée d'un cycle complet est typiquement de moins de 15 secondes. Toutefois, la durée de la phase de pause inspiratoire n'excède pas 1 seconde, en général elle est inférieure à 800 msec, voire même préférentiellement inférieure à 600 msec.

Par ailleurs, la pression réglé (Pr) est typiquement comprise entre 5 et 70 cmH2O.

Comme illustré en Fig. 1, l'appareil d'insufflation et d'exsufflation 1 selon l'invention comprend un générateur de flux 2 servant à insuffler et/ou exsuffler du gaz pendant un ou des cycles INEX successifs mis en oeuvre dans le cadre d'une thérapie INEX.

De préférence, le générateur de flux 2 est ou comprend une turbine motorisée 2.1, aussi appelée compresseur, (micro-)soufflante ou analogue. En outre, le générateur de flux peut aussi comprendre une turbine associée à des (électro)vannes et/ou valves pneumatiques.

Le générateur de flux 2, c'est-à-dire la turbine 2.1, est accordé fluidiquement à un circuit de gaz 3, 4, 8 comprenant une ligne d'insufflation 3 de gaz pour acheminer le gaz, à savoir ici de l'air, délivré par le générateur de flux 2, pendant chaque phase d'insufflation de gaz ; et une ligne d'exsufflation 4 pour acheminer du gaz jusqu'au générateur de flux 2, pendant au moins une phase d'exsufflation de gaz, à savoir le gaz ressortant des voies aériennes du patient (i.e. les gaz expirés contenant du CO₂). En répétant ces phases sur plusieurs cycles d'affilée, on peut mimer le mécanisme de la toux.

Les lignes d'insufflation 3 et d'exsufflation 4 viennent de raccorder fluidiquement à un tronçon de jonction 8 commun du circuit de gaz 3, 4, 8, c'est-à-dire un conduit commun ou analogue, en communication fluidique, via un tuyau ou conduit flexible 12, avec une interface respiratoire 10, tel un masque respiratoire facial agencé sur le visage du patient, c'est-à-dire recouvrant sur son nez et sa bouche. Alternativement, on peut aussi utiliser, selon le cas, un embout buccal, une sonde de trachéostomie ou analogue.

Le tronçon de jonction 8 commun est situé à l'extrémité aval du circuit de gaz 3, 4, 8 comprenant sa sortie aval 14.

Le tuyau ou conduit flexible 12 vient se raccorder fluidiquement à des moyens de raccordement 13 situés au niveau de la sortie aval du circuit de gaz 3, 4, 8.

Les lignes d'insufflation 3 et d'exsufflation 4, et le tronçon de jonction 8 commun sont agencés principalement dans l'appareil 1, c'est-à-dire dans la carcasse ou coque périphérique de l'appareil. Ils peuvent comprendre des conduits de gaz, des passages ou analogues.

Durant les phases d'insufflation, le gaz chemine dans le tronçon de jonction 8 en direction du patient, alors que durant les phases d'exsufflation, le gaz y chemine dans le sens opposé, c'est-à-dire en direction du générateur de flux 2, i.e. la turbine 2.1.

Autrement dit, la ligne d'insufflation 3 relie fluidiquement une sortie de la turbine 2.1 à l'interface respiratoire 10, via le tronçon de jonction 8 et le tuyau flexible 12, afin de permettre de fournir au patient, l'air délivré par la turbine 2.1 durant les phases d'insufflation, alors que la ligne d'exsufflation 4 relie fluidiquement l'interface respiratoire 10, via le tuyau flexible 12, à une entrée de la turbine 2.1 de manière à pouvoir aspirer le gaz ressortant des voies aériennes du patient (aussi appelé gaz « expiré » riche en CO₂), durant les phases d'exsufflation.

L'appareil d'insufflation et d'exsufflation 1 comprend en outre des moyens de contrôle de flux 5 agencés sur ledit circuit de gaz 3, 4, 8, permettant de contrôler la circulation du (des) gaz dans ledit circuit de gaz 3, 4, 8, typiquement dans (au moins) les lignes d'insufflation 3 et d'exsufflation 4, durant les cycles INEX successifs, à savoir pendant les phases d'insufflation et d'exsufflation de gaz, et une ou des phases de pause éventuelle(s).

De préférence, les moyens de contrôle de flux 5 comprennent une ou des vannes pilotées, typiquement des électrovannes et, de préférence, des électrovannes associées à des valves pneumatiques. Un exemple d'appareil d'insufflation et d'exsufflation à un système de contrôle de flux incluant une turbine motorisée, des électrovannes et des valves pneumatiques est décrit par EP3622991, auquel on peut se reporter.

Par ailleurs, sont prévus aussi des moyens de pilotage 6 à électronique de commande, c'est-à-dire comprenant un ou plusieurs microprocesseurs, microcontrôleurs ou analogues. Typiquement une (ou des) carte électronique sur laquelle est (sont) agencé le (les) microprocesseur(s) ou microcontrôleur(s) qui met en oeuvre un ou des algorithmes de traitement de mesures ou autres, de pilotage, d'affichage ou autres.

Ainsi, les moyens de pilotage 6 sont configurés pour assurer un bon fonctionnement de l'appareil d'insufflation et d'exsufflation 1, notamment :
- pour piloter le générateur de flux 2, en particulier du moteur électrique de la turbine 2.1 motorisée durant les cycles INEX ;
- pour commander les moyens de contrôle de flux 5, typiquement des électrovannes, de préférence associées à des valves pneumatiques, pour autoriser ou empêcher les circulations des flux gazeux dans tout ou partie du circuit de gaz 3, 4, 8, en particulier dans les lignes d'insufflation 3 et d'exsufflation 4 ;
- pour gérer les affichages sur l'écran d'affichage 11, comme expliqué ci-après.

Autrement, les moyens de pilotage 6 permettent de contrôler le générateur de flux 2, pendant les cycles INEX successifs, chaque cycle INEX comprenant une phase d'insufflation de gaz et une phase d'exsufflation de gaz, et éventuellement phase de pause respiratoire entre au moins une phase d'insufflation de gaz et une phase d'exsufflation de gaz ; et/ou les moyens de contrôle de flux 5 pour autoriser ou interdire les circulations des flux gazeux dans les lignes d'insufflation 3 et d'exsufflation 4 en fonction du déroule de chaque cycle INEX, c'est-à-dire pendant les phases d'insufflation (in) de gaz et d'exsufflation (ex) de gaz, et éventuellement de pause respiratoire, c'est-à-dire de gérer les phases in-ex et par ailleurs celle de pause.

Par ailleurs, l'appareil d'insufflation et d'exsufflation 1 comprend aussi des moyens de mesure de pression 7, typiquement un (des) capteur de pression, pour mesurer la pression du gaz dans le circuit de gaz 3, 4, 8, en aval 9 des moyens de contrôle de flux 5 et pour ensuite fournir la (les) mesure de pression aux moyens de pilotage 6 qui les traitent afin de déterminer la pression qui règne dans la partie de circuit de gaz 3, 4, 8 située en aval 9 des moyens de contrôle de flux 5.

Bien entendu, sont aussi prévus des moyens d'alimentation électrique, telle une liaison au secteur (110/220V), i.e. câbles et prise de raccordement, et/ou une (des) batterie interne rechargeable, pour alimenter tous les composants de l'appareil 1 nécessitant de la puissance électrique pour fonctionner, notamment les moyens de pilotage 6, l'écran d'affichage 11, les moyens de contrôlent de flux 5, i.e. les électrovannes pilotées...

En particulier, selon l'invention, afin de pouvoir améliorer le traitement INEX du patient en détectant les phénomènes indésirables de type fuites au niveau de l'interface respiratoire (e.g. masque), fermeture de glotte, réglage de pression inadapté ou autres, susceptibles de nuire à l'efficacité d'une thérapie INEX, les moyens de pilotage 6 sont en outre configurés pour :
- déclencher une phase de pause respiratoire à la fin d'au moins une phase d'insufflation en contrôlant les moyens de contrôle de flux 5 pour stopper ou empêcher toute circulation de gaz au sein desdites lignes d'insufflation 3 et d'exsufflation 4, c'est-à-dire bloquer la circulation de gaz dans les deux lignes 3, 4 et ainsi isoler la partie aval du circuit de gaz 3, 4, 8 du reste du circuit qui est, de préférence, maintenu sous pression.
- comparer la pression de pause (Pp) mesurée par les moyens de mesure de pression 7, pendant ladite pause respiratoire opérée, à une pression de référence (Pr) fixée par l'utilisateur, et préférentiellement mémorisée. Les mesures de pression de pause (Pp) sont opérées dans la partie aval du circuit de gaz 3, 4, 8 qui comprend des portions de circuit situées entre les moyens de contrôle de flux 5, e.g. les électrovannes pilotées, et le site de raccordement fluidique 13 du tuyau flexible 12. Sur Fig. 1, ces portions de circuit comprennent notamment des portions aval 3.1 et amont 4.1 des lignes d'insufflation 3 et d'exsufflation 4, et le tronçon commun 8.
- et détecter l'existence d'un phénomène indésirable lorsque la pression de pause (Pp) mesurée au sein de ces portions de circuit est inférieure à la pression de référence (Pr) ou à une proportion de ladite pression de référence (Pr) fixée par l'utilisateur, c'est-à-dire Pp < x%.Pr (avec x% compris entre 50% et 95%). La valeur de seuil (x%) peut être réglable ou réglée par l'utilisateur.

En cas de détection d'un phénomène indésirable limitant les performances du traitement INEX, typiquement empêchant un bon remplissage des voies aériennes du patient, les moyens de pilotage 6 peuvent avertir l'utilisateur de la détection de ce phénomène indésirable, par exemple en commandant un affichage d'une alerte sur les moyens d'affichage 11 de l'appareil 1, typiquement un écran tactile, c'est-à-dire à dalle tactile.

Dans le cadre de l'invention, les phénomènes indésirables sont ou comprennent principalement une ou des fuites gazeuses au niveau de l'interface respiratoire, c'est-à-dire un défaut d'étanchéité du masque ou analogue ; une fermeture intempestive de la glotte du patient pendant l'insufflation ; et/ou un temps inspiratoire ayant été réglé qui est trop court pour permettre d'une insufflation efficace ou suffisante de gaz.

L'affichage de l'alerte sur les moyens d'affichage 11 de l'appareil 1 peut revêtir différentes formes comme un affichage d'une information ou d'une alerte de détection, par exemple un message d'avertissement alphanumérique ou autre, par exemple un code d'alerte ; une animation vidéo ; une icône ou fenêtre d'alerte dédiée ou autre. L'affichage de l'information ou de l'alerte de détection peut aussi se matérialiser sous forme d'un niveau ou proportion de baisse de pression (i.e. un pourcentage, i.e. %) ou de toute autre valeur ou grandeur permettant d'exprimer la perte de performance

Etant alerté quant à l'existence d'un (ou plusieurs) phénomène indésirable, l'utilisateur, c'est-à-dire le personnel soignant, peut intervenir pour corriger le problème engendrant le phénomène indésirable, par exemple ajustant ou appuyant sur le masque pour améliorer son étanchéité, augmenter le temps insufflatoire et/ou réduire la pente inspiratoire ou la pression inspiratoire de manière à éviter ou minimiser la fermeture glottique.

Selon un mode de réalisation, en plus de l'existence d'un (ou plusieurs) phénomène indésirable, il peut être souhaitable de pouvoir différencier entre les différentes causes de phénomène indésirable.

Pour ce faire, dans l'appareil d'insufflation et d'exsufflation de gaz 1 selon l'invention, les moyens de pilotage 6 à une électronique de commande peuvent être configurés pour analyser la forme du signal de pression pendant la phase de pause inspiratoire et en déduire une (des) cause possible de phénomène indésirable.

Ainsi, si les moyens de pilotage 6 déterminent ou constatent un signal de pression qui chute et qui reste stable pendant la durée de la pause inspiratoire, comme illustré sur la Fig. 2, ils vont en déduire un problème de fermeture glottique ou de remplissage incomplet des voies aériennes du patient. De manière plus précise, les moyens de pilotage 6 peuvent réaliser aussi une analyse de la courbe de débit au cours de la phase inspiratoire avant la pause. S'il est mis en évidence que le débit a chuté brutalement au cours de l'insufflation, il est probable que la cause soit une fermeture glottique, alors qu'en revanche, si le débit est encore significatif avant le début de la phase de pause, la cause est plus vraisemblablement un remplissage incomplet des voies aériennes du patient.

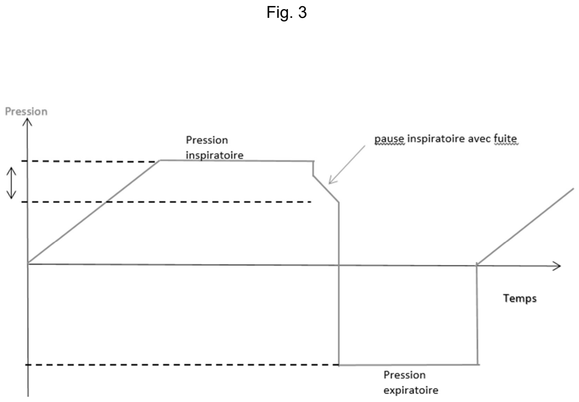

Par ailleurs, si les moyens de pilotage 6 déterminent ou constatent un signal de pression qui chute plus progressivement, comme illustré sur la Fig. 3, ils en déduisent que la cause est une fuite car, dans ce cas, la perte de pression est progressive sur toute la phase de pause inspiratoire.

Par exemple, une chute de pression minimale de 0.1 mb à 20 mb (ou bien entre 1% et 50% de la pression d'insufflation) par 100 msec entre le début (après environ 20 à 50 msec de stabilisation) et la fin de la pause inspiratoire peut constituer un seuil au-delà duquel le phénomène indésirable est considéré comme une fuite.

Le seuil peut être adaptatif en fonction de différentes variables comme le volume insufflé, la pression d'insufflation réglée, le temps d'insufflation, la chute initiale de la pression sur les 20 à 50 premières msec de la pause inspiratoire, ou tout autre réglage et/ou monitorage ayant une influence sur la chute de pression associée à une fuite.

Ensuite, les moyens de pilotage 6 peuvent être configurés pour indiquer à l'utilisateur la présence de fuites ou d'un autre type ou cause de phénomènes indésirables suivant une analyse du signal de pression pendant la phase de pause inspiratoire, voire du signal de débit, typiquement en affichant sur les moyens d'affichage 11, au moins une cause possible d'un (ou plusieurs) phénomène indésirable ayant été identifié.

L'appareil 1 de l'invention est bien adapté au traitement des patients (i.e. enfants, adolescents, adultes...) présentant un encombrement et une faiblesse musculaire, par exemple des patients ayant une maladie neuromusculaire lente à progressive et/ou aux patients paralytiques d'étiologie traumatique.

## Revendications

1. Appareil d'insufflation et d'exsufflation de gaz (1) comprenant :
- un générateur de flux (2) configuré pour insuffler et/ou exsuffler du gaz,
- un circuit de gaz (3, 4, 8) comprenant une ligne d'insufflation (3) pour acheminer du gaz délivré par le générateur de flux (2), pendant au moins une phase d'insufflation de gaz, et une ligne d'exsufflation (4) pour acheminer du gaz jusqu'au générateur de flux (2), pendant au moins une phase d'exsufflation de gaz, lesdites lignes d'insufflation (3) et d'exsufflation (4) étant raccordées fluidiquement à un tronçon de jonction (8) commun configuré pour être raccordé fluidiquement (en 13) à un conduit flexible (12) alimentant une interface respiratoire (10),
- des moyens de contrôle de flux (5) agencés sur ledit circuit de gaz (3, 4, 8), configurés pour contrôler la circulation du gaz dans au moins lesdites lignes d'insufflation (3) et d'exsufflation (4),
- des moyens de pilotage (6) comprenant une électronique de commande configurés pour contrôler le générateur de flux (2) et les moyens de contrôle de flux (5) et
- des moyens de mesure de pression (7) configurés pour mesurer la pression du gaz dans le circuit de gaz (3, 4, 8) entre les moyens de contrôle de flux (5) et le site de raccordement fluidique (13) au conduit flexible (12), et fournir ladite au moins une mesure de pression aux moyens de pilotage (6),
**caractérisé en ce que** les moyens de pilotage (6) sont en outre configurés pour :
a) déclencher une phase de pause respiratoire à la fin d'au moins une phase d'insufflation en contrôlant les moyens de contrôle de flux (5) pour stopper ou empêcher toute circulation de gaz au sein desdites lignes d'insufflation (3) et d'exsufflation (4),
b) comparer au moins une pression de pause (Pp) mesurée par les moyens de mesure de pression (7) pendant ladite pause respiratoire, à une pression de référence (Pr) fixée par l'utilisateur, et
c) détecter l'existence d'un phénomène indésirable, lorsque ladite au moins une pression de pause (Pp) mesurée est inférieure à la pression de référence (Pr) ou à une proportion de ladite pression de référence (Pr) fixée par l'utilisateur.

2. Appareil selon la revendication 1, **caractérisé en ce que** le générateur de flux (2) comprend une turbine (2.1) motorisée, de préférence la turbine (2.1) motorisée délivre un flux d'air.

3. Appareil selon la revendication 1, **caractérisé en ce que** le tronçon de jonction (8) est situé à une sortie (14) du circuit de gaz (3, 4, 8).

4. Appareil selon la revendication 1, **caractérisé en ce que** la pause respiratoire a une durée de pause comprise entre 100 msec et 1 seconde, de préférence entre 150 et 400 msec.

5. Appareil selon la revendication 1, **caractérisé en ce que** les moyens de pilotage (6) sont configurés pour détecter un phénomène indésirable lorsque ladite au moins une pression de pause (Pp) mesurée est inférieure ou égale à 95% de la pression de référence (Pr) fixée par l'utilisateur, typiquement une valeur de seuil (x%) comprise entre 50 et 90%.

6. Appareil selon la revendication 1, **caractérisé en ce que** les moyens de pilotage (6) sont en outre configurés pour avertir l'utilisateur de la détection d'un phénomène indésirable.

7. Appareil selon la revendication 6, **caractérisé en ce qu'**il comprend un écran d'affichage (11) et les moyens de pilotage (6) sont configurés pour contrôler ou opérer un affichage d'une information ou alerte de détection de phénomène indésirable sur ledit écran d'affichage (11).

8. Appareil selon la revendication 1, **caractérisé en ce que** les moyens de contrôle de flux (5) comprennent une ou plusieurs vannes, typiquement une ou des électrovannes, de préférence associées à une ou des vannes pneumatiques.

9. Appareil selon la revendication 1, **caractérisé en ce qu'**il comprend des moyens de réglage de pression configurés pour permettre à l'utilisateur de régler ou fixer ladite pression de référence (Pr).

10. Appareil selon la revendication 1, **caractérisé en ce que** les phénomènes indésirables sont choisis parmi une fuite gazeuse, une fermeture de la glotte du patient et un temps inspiratoire réglé trop court.

11. Appareil selon la revendication 5, **caractérisé en ce qu'**il comprend des moyens de réglage de valeur de seuil (x%) configurés pour permettre à l'utilisateur de régler ou de fixer une valeur de seuil (x%) comprise entre 50% et 90% de ladite pression de référence (Pr).

12. Appareil selon la revendication 1 et 11, **caractérisé en ce qu'**il comprend des moyens de mémorisation et/ou les moyens de pilotage (6) sont configurés pour mémoriser au sein des moyens de mémorisation, la valeur de pression et/ou la valeur de seuil (x%) réglée ou fixée.

13. Appareil selon la revendication 1 ou 9, **caractérisé en ce que** la pression réglée (Pr) est comprise entre 5 et 70 cmH20.

14. Appareil selon la revendication 1, **caractérisé en ce que** les moyens de pilotage (6) sont configurés pour commander, contrôler ou piloter le générateur de flux, pendant un ou des cycles successifs d'insufflation et d'exsufflation de gaz (INEX), chaque cycle INEX comprenant au moins une phase d'insufflation de gaz et une phase d'exsufflation de gaz, la durée d'un ou de chaque cycle INEX étant inférieure ou égale à 15 secondes.

15. Appareil selon la revendication 1, **caractérisé en ce que** les moyens de pilotage (6) sont en outre configurés pour analyser la forme d'un signal de pression pendant la phase de pause inspiratoire et en déduire au moins une cause possible de phénomène indésirable.
